# EUROPEAN PATENT SPECIFICATION

(11) **EP 1 755 726 B1**
(45) Date of publication and mention of the grant of the patent: **23.12.2015**
(21) Application number: 05735515.8
(22) Date of filing: 16.04.2005
(51) Int. Cl.: A61M 29/00

(54) **ASYMMETRICAL MEDICAL FILTER**
ASYMMETRISCHER MEDIZINISCHER FILTER
FILTRE MEDICAL ASYMETRIQUE

(30) Priority: 16.04.2004 US 563058 P
(43) Date of publication of application: 28.02.2007
(73) Proprietor: Cordis Corporation, Miami Lakes, Florida 33014 (US)
(72) Inventor: SULLIVAN, Jason, R., Lebanon, NJ 08833 (US); WONG, Wai, Chung, Jonathan, Chino Hills, California 91709 (US)
(74) Representative: Belcher, Simon James
(86) International application number: PCT/US2005/012877
(87) International publication number: WO 2005/102437

(56) References cited:
- WO-A-95/09567
- US-A- 4 425 908
- US-B1- 6 241 746
- US-B1- 6 364 895
- US-B1- 6 443 972

## Description

### BACKGROUND AND SUMMARY OF THE INVENTION

### 1. Technical Background:

The present invention relates to a medical filter.

### 2. Discussion:

Some basic types of medical filters are generally known, wherein a single filter element, mesh or member spans a body passage, or extends across the direction of flow inside a blood vessel. The medical filters of the present invention will generally be described in a vascular setting, though it should be understood that the present invention may be positioned to treat other types of body passages, including for example the biliary system.

Several features may be desirable for medical filters, including non-surgical or "percutaneous" delivery of the filter to a desired site, and expansion from a preferably small initial size to an expanded working size that matches the anatomy at the desired site.

In the case of a vascular filter, it should preferably capture a sufficient percentage of thrombus, while allowing blood to flow freely through the filter. Another desirable feature is a capability to remain reliably in the desired position in a patient's anatomy, referred to as "position retention." In addition, a vascular filter should preferably have a design whereby the filter is self-centering and stable in the vessel, such that the filter has a tendency not to "tilt", which might result in less effective capturing of thrombus. Some vascular filters may be used in the vena cava, and may be described in such event as a "vena cava filter."

A vascular filter may be delivered through a catheter in a compressed shape, the filter tending to resiliently expand within a blood vessel and to retain the desired position and orientation. The vascular filter tends to trap thrombus or particles, and resist their movement further downstream. The filter includes, in a position of use, an outer shape corresponding to the internal diameter of the blood vessel, and one or more filter elements extending across the vessel.

Some known vascular filters have a double-basket design with a symmetrical "flower" or diamond pattern on each end. An advantage of such filters is that clots can be captured in both baskets. To increase filter efficiency and resist a possibility of occlusion that may exist, the filter may preferably be designed to capture clot material in each basket with approximately equal distribution. In other words, it may be desirable to design such a double-basket filter so that any amount of thrombus that may be captured in each of the filter baskets tends to be about equal.

In some prior double-basket filters, a higher percentage of thrombus might be captured in the caudal basket. The reason is that this is the "upstream" basket, or the first basket encountered by the flow of fluids and any particulates. This disparity in collection among baskets may result in: (i) some degree of increased obstruction, as more clot material is captured in one basket than the other basket; (ii) decreased fluid flow through the filter, due to preferential accumulation of contents in one basket; and (iii) upstream flow drag, that may induce thrombosis in the cranial basket if the caudal basket reaches its capacity.

US-6443972 discloses a vascular filter which can be placed in a blood vessel and which comprises at least one passage with a circumference suitable for the purposes of intercepting a thrombus. The filter comprises a longitudinal body member which in use has a circumference corresponding to the internal diameter of the blood vessel transverse to the longitudinal direction of the blood vessel. The filter has first and second filter sections which are symmetrical.

WO-95/09567 discloses a vascular filter comprising an emboli catching portion having a set of helical filter wires joined at a central region and extending in a given direction along the blood vessels in a diverging relationship.

US-4425908 discloses a blood clot filter which is inwardly radially compressible for insertion into a vein and automatically radially expandable within the vein. In the expanded configuration the filter comprises a plurality of wires in the form of overlapping loops with portions of the loops contacting the inner wall of the vein providing a filter basket at the leading end of the filter.

According to the present invention there is provided a medical filter for therapeutic treatment of a patient, according to claim 1.

According to the principles of the present invention, it is possible to achieve the desired effect of the second filter section exhibiting a greater filtering efficiency than the first filter section by providing a first and second filter basket having disparate filtering capabilities. For example, the second or "downstream" filter basket may be designed having a greater relative filter efficiency than the first or "upstream" filter basket.

Of course, there are a variety of ways in which this tailored filter basket performance can be achieved. Some examples include increasing the number of struts or decreasing the size of the filter cells or other filter elements of the second or "downstream" filter basket. Likewise, a similar result may be reached by decreasing the number of struts or increasing the size of the filter cells or other filter elements of the first or "upstream" filter basket.

In one particular example shown in the drawings, asymmetrical filter basket "flower" patterns are provided by reducing the number of segments originating at the first "upstream" end, which may also be referred to as the caudal collar, from six to three, then bifurcating these segments to create the six middle straight struts that touch the vessel wall. This particular configuration retains the longitudinally extended, double basket arrangement that provides for minimal tilting during deployment, while eliminating the symmetry between ends of the filter.

As a result, the filtration pattern of the first "upstream" or caudal basket is slightly reduced, thereby reducing the clot capturing efficiency of that basket. Consequently, less thrombus tends to be captured in the first caudal basket, and is instead more likely to be captured in the second cranial basket. This disparate filtering efficiency optimizes the dual basket design to maintain adequate levels of filtration while reducing the factors which might contribute to occlusions.

Further, this partial shift in filtering or clot capturing from the first caudal basket to the second cranial basket may also increase the total clot burden tolerance (i. e. total mass of thrombus captured before occlusion or thrombosis) and increase the successful thrombus lysis rate due to the converging geometry of the cranial basket and its tendency to trap clot in the center of the vessel lumen.

In the temporal sense, there are three types of filters: (i) permanent filters, intended for permanent implantation; (ii) temporary filters, intended for removal within a time period; and (iii) retrievable filters, in which the physician has the option to implant the filter permanently or to remove the filter after some time. In the case of a retrievable filter, the filter may be designed so that the physician can choose whether to retrieve the filter at a later date, after the filter has been in place for a while. This way, the physician can evaluate the performance of the filter and the patient's condition, before deciding whether to retrieve the filter or not.

Regarding retrieval, one factor is "endothelialization" or in-growth of the vessel wall and tissue around the structural members of the filter. In other words, endothelialization is the healing of the vessel inner surface by endothelial cells, and it is desirable to preserve these endothelial cells when removing a retrievable vascular filter. The improved designs of the present invention tend to minimize any impact during retrieval.

Prior vascular filters have a demonstrated track record of filtering clots due to their filter basket design. However, because of the tendency of neointimal tissue to grow over the struts and other filter elements, may cause the filter to become mechanically interlocked with the tissue. When the tissue develops sufficient strength, the filter may no longer be retrievable. For some prior retrievable filters, this may occur somewhere between two and three weeks following implantation.

The filters shown in the drawings are both retrievable vascular filters and permanent vascular filters. One factor in determining the duration of retrievability is the extent of intimal ingrowth of the vascular lining over the filters' struts, which may result in physical incorporation. The geometry of the filters shown in the drawings are such that initial incorporation occurs at the transition from the flower patterns to the middle straight struts that touch the vessel wall. This incorporation occurs because the segments (considering a caudal-to-cranial perspective) defining the filter converge at the transition point on the first or caudal end of the filter (confluence), and diverge at the transition point on the second or cranial end of the filter (bifurcation). Once tissue has overgrown these confluences and bifurcations, the filter segments are incorporated and cannot be withdrawn from the tissue as easily as prior to this overgrowth.

Theoretically, a bifurcation may be more easily withdrawn from tissue than a confluence because the bifurcation tends to separate the tissue under tension when the filter is pulled in the caudal direction. Alternatively, a confluence tends to pile together the tissue under compression when the filter is pulled in the caudal direction. Thus, for the same amount of tissue ingrowth, the bifurcation may require less force to withdraw from its entrapment than the confluence. Conversely, for the same amount of withdrawal force, the bifurcation may be withdrawn from greater depth of entrapment in tissue than the confluence. This would equate to increased duration of retrievability for a retrievable vascular filter, as the process of intimal ingrowth is time dependent.

The filters shown in the drawings eliminate the confluence created at this transition point of the caudal end of the filter because the geometry forms the six middle straight struts from only three segments originating at the caudal collar. Thus, the filter geometry (considering a caudal-to-cranial perspective) still has only nine bifurcations rather than twelve, and only six confluences rather than twelve (not including the point of origination of the segments at the caudal collar and the termination of the segments at the cranial collar). This would serve to decrease the resistance to removal for a given thickness of tissue ingrowth and equate to an increased duration of retrievability.

A vascular filter along the lines of the present invention may provide several advantages, including effectively capturing thrombus while allowing blood flow, tending to capture approximately equal amounts of thrombus, and extending the retrievability period of the filter.

The vascular filter of the present invention has an initial compressed shape, in which the filter may have essentially a tubular shape, and may be contained in a lumen or passage defined by a catheter. After a distal tip of the catheter reaches a desired site for treatment, a wire mandrel or other deployment device may be used to push the filter out of the catheter. And when the filter is released from the catheter, it tends to resiliently expand from the initial compressed shape to an expanded shape. When a vascular filter is retrieved from a blood vessel, the entire filter is resiliently compressed to a relatively small diameter, for extraction through a catheter.

The term "tubular" is used in its broadest sense, to encompass any structure arranged a radial distance around a longitudinal axis. Accordingly, "tubular" includes any structure that (i) is cylindrical or not, such as for example an elliptical or polygonal cross-section, or any other regular or irregular cross-section; (ii) has a different or changing cross-section along its length; (iii) is arranged around a straight, curving, bent or discontinuous longitudinal axis; (iv) has an imperforate surface, or a periodic or other perforate, irregular or gapped surface or cross-section; (v) is spaced uniformly or irregularly, including being spaced varying radial distances from the longitudinal axis; (vi) has any desired combination of length or cross-sectional size.

The vascular filter of the present invention includes a first and second filter section, arranged on either side of a body section. The body section and the filter sections thus enclose a space. Due to the elongated shape of the vascular filter, and the arranging of the first and second filter sections on either side of the body member, the present filter has an enhanced filtering effect. In other words, two opportunities have been created for intercepting thrombus moving inside the blood vessel.

A central tubular section tends to resiliently exert slight outward pressure along a large section of contact area on the blood vessel wall. The sleeve distributes this outward pressure to a greater area. Accordingly, the filter tends to exert some small amount of pressure on the internal wall of the blood vessel, and tends to hold itself in place. The vascular filter will consequently tend not to shift position. In addition, because of this elongated shape the vascular filter tends to center itself within the lumen, and not to rotate transversely or tilt over.

In an example, a vascular filter may be formed out of one single piece, which provides advantages including simplicity.

When viewed along the longitudinal axis of the filter, the filter sections may have the shape of a regular polygon, and thus may provide several smaller filtering "cells". The purpose of these filtering cells is to intercept thrombus moving inside the blood vessel, and the smaller filtering cells tend to capture more thrombus. All the cells may be of the same size, to provide a uniform filtering effect.

It is of course possible to build various vascular filters according to the present invention, by various techniques and of various materials to obtain the desired features.

These and various other objects, advantages and features of the invention will become apparent from the following description and claims, when considered in conjunction with the appended drawings. The invention will be explained in greater detail below with reference to the attached drawings of a number of examples of embodiments of the present invention.

### BRIEF DESCRIPTION OF THE DRAWINGS

Figure 1 illustrates a side elevation view of a medical filter;
Figure 2 shows a transverse cross-section view of a medical filter;
Figure 3 shows a transverse cross-section view of a medical filter;
Figure 4 shows a side elevation view of another medical filter;

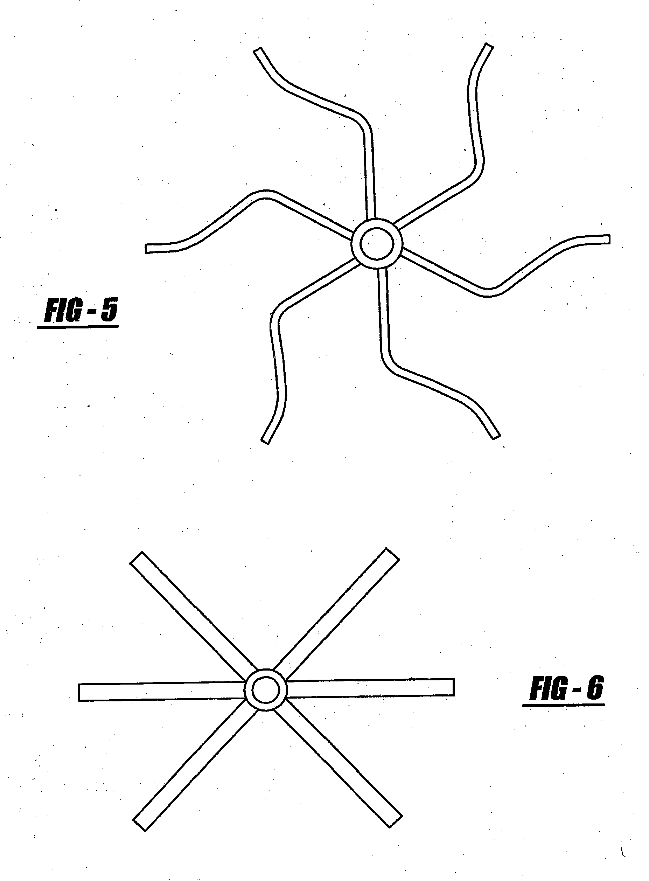

Figure 5 shows a transverse cross-section view of a medical filter; and
Figure 6 shows a transverse cross-section view of a medical filter.

### DETAILED DESCRIPTION OF THE PREFERRED EMBODIMENTS

The following description of the preferred embodiments of the present invention is merely illustrative in nature, and as such it does not limit in any way the present invention, its application, or uses. Numerous modifications may be made by those skilled in the art without departing from the scope of the invention.

The drawings depict vascular medical filters along the lines of the present invention.

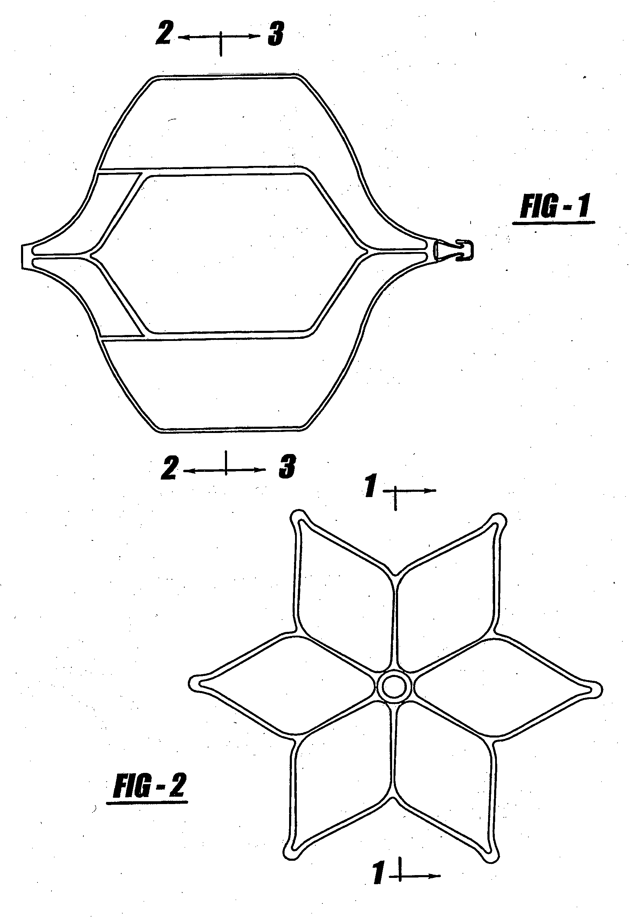

The designs shown in Figures 1-4 are based on the dual-basket construction of the successful Cordis OptEase™ filter. The second or "downstream" cranial basket remains unchanged, and the flower pattern may be removed from the first or "upstream" caudal basket by alternately eliminating three of the segments originating at the caudal collar. These three segments would then bifurcate and transition to the middle straight struts. Both modifications open up the caudal basket and allow clots to be more likely trapped in the cranial basket.

The filter designs of the present invention may include the following advantages: (i) reducing thrombosis or occlusion at the caudal basket by decreasing the quantity of clot captured by the caudal basket, or decreasing the amount of implant material that may induce turbulence; (ii) reducing thrombosis or occlusion at the cranial basket by decreasing turbulence induced by clot captured in the caudal basket, or opening up the caudal basket and minimizing the "up-stream" flow resistance; (iii) increasing preferential capturing of clots in cranial basket where the clots are directed to the center of lumen, which may result in reducing a tendency for clots to organize and attach to the vessel wall, or increasing the lysis rate due to flow dynamics of centrally captured clot. Increasing the likelihood of clot being captured in interior of filter may increase the retrievability of the filter because clot trapped inside a filter is more likely to be retrieved with the filter due to entrapment, rather than stripped away from the filter during filter collapse and retrieval. Another advantage may include: (iv) increasing retrievable period by alterations of the filter geometry by eliminating three bifurcations in the caudal flower pattern, or eliminating six confluences at the transition from the caudal segments to the straight middle segments, or increasing the ratio of bifurcations to confluences.

As shown in the drawings, the filter has an expanded shape, and an initial compressed shape. If the filter is delivered with a catheter and a pushing wire or mandrel, the filter will have the initial compressed shape when it is within a passage or lumen of the catheter. In this configuration, the filter may have a tubular shape, and a pattern of struts or ribs may be affixed together or be made of a single piece of material with a series of cuts.

In any event, the filter tends to resiliently expand from the initial compressed shape to the expanded shape. Once the filter is in the expanded shape, it tends to resiliently maintain that expanded shape, when deployed at a desired site for treatment within a body passage or vessel.

Filters of the present invention may be made with various manufacturing methods, including providing an initial tube, and then cutting a series of struts in the tube to enable expansion into the desired shape. Various other methods are of course possible, including forming the filter of discrete members and joining or connecting the members, or chemically etching a substrate. The manufacturing methods may include an inflatable or expandable mold, heating or cooling, welding, etc.

To clarify one possible method of making a filter, an initial tubular form defines a longitudinal axis and has first and second ends. More than one strut is cut in the tubular form, so as to define struts extending between the first and second end; and the struts are treated so that they tend to resiliently expand from a compressed shape to an expanded shape. A central portion of each of the struts is expanded in a radially outward direction, such that a gap is defined between the struts. As shown in the drawings, in the expanded shape, the struts define a first and second filter section and a central section connecting the first and second filter sections.

Structurally, when viewed from the side, a filter in the expanded deployed shape has a central section, flanked by a first and second filtering section, which are flanked by a first and second end. The particular examples depicted in the drawings is made from a single piece of tubular material, with a patterned series of cuts, which is treated to resiliently expand and form the filtering mesh structure. The filter structure could of course also be formed of multiple members which are affixed together.

The terms "filter" or "vascular filter" or "filtering" may be used in a broad or interchangeable fashion to refer generally to the entire the filter, the first and second filtering section, the filtering effect on body fluids or particulates, or the results of such a filtering effect, or any other relevant aspect of the present invention.

While the filter is implanted within a patient, body tissues naturally tend to incorporate or endothelialize implanted objects. This process of endothelialization may take place over a predictable period of time, and when a filter or other medical device has been incorporated or endothelialized, it may be preferred to leave it in place indefinitely.

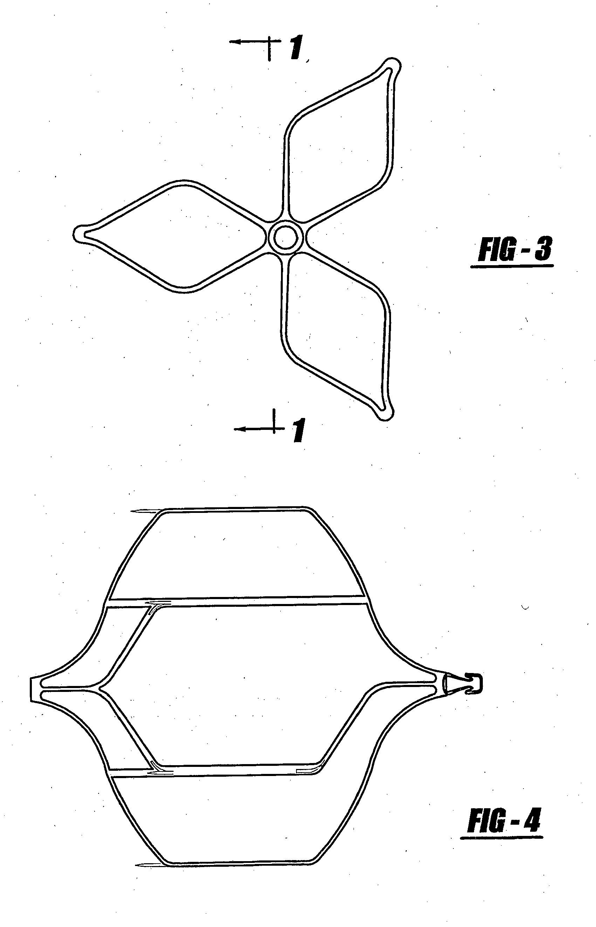

If the filter is intended to be a temporary or a retrievable filter, such that the filter may be removed or retrieved at a later time, the filter may be provided with features advantageous to such possible retrieval. For example, the filter shown in Figure 4 has some additional optional features, that may be included but are not required in a filter arranged according to the present invention, including a hook structure, barbs or anchors, and apertures. Barbs may be desirable to provide releasable temporary position stabilizers, to resist tilting and to enhance position retention. The hook may be used to extract the filter back into a catheter by means of a cooperating hook, snare or grabbing member.

In the compressed shape when the vascular filter is inside the catheter, the filter may include cuts extending in the longitudinal direction of the filter between, but not as far as, the ends of the filter. The cuts define strips of material as illustrated in the drawings. These strips expand to form the filtering first and second mesh, and the ribs. The specific cuts consequently also form the filter elements on either side of the filter. The strips extend in a generally longitudinal direction in the compressed shape.

The vascular filter embodiment illustrated here may of course be used in the vena cava or any other desired site for treatment. The filter includes a number of ribs or struts extending in a generally undulating longitudinal direction. Liquid inside the blood vessel can pass through the vascular filter, but thrombus or particulates tend to be intercepted by one of the two filter sections.

In the axial views of Figures 1 and 2, the filter sections on either side of the ribs of the vascular filters according to the present invention described above may display diamond or polygon shapes. It is also possible to provide vascular filters of which the filter sections display in axial view a star shape, or any other suitable shape, as long as they successfully intercept blood clots or thrombus. An advantage of this feature is that, after passing the first filter section and the tubular section or the elongated body member, a second filter element for intercepting thrombus has been provided. Also, other shapes of the filter sections in axial view are possible, which shapes will occur to those skilled in the field after reading the present description. The shapes of the filter sections in axial view need not be symmetrical, and may in principle have any suitable appearance.

Furthermore, retraction of a vascular filter according to the present invention is mentioned above, which should not limit the scope of the claims attached. Regarding the subject of the invention, it is therefore of no consequence whether the filter is placed permanently, in a removable manner, temporarily or otherwise.

Vascular filters according to the present invention may be made of any suitable material using a variety of methods. One material having the desired characteristics of strength, resilience, flexibility, biocompatibility and endurance is nitinol. Other possible materials include stainless steel and any other material having the desired properties.

Some possible modifications may include: (i) as opposed to three segments originating at the caudal collar which in turn bifurcate into the six middle straight struts, rather an alternative embodiment could consist of six segments originating at the caudal collar which transition directly into the six middle straight struts; (ii) the location of the transition from three struts to six struts can be varied to produce the maximum retrieval period; or (iii) the edges of the segments at the vertexes of the confluences could be shaped or sharpened to reduce the resistance to removal and thereby increase the retrievability period.

Dual hooks could be included with the vascular filter of the present invention to facilitate dual direction retrieval.

It should be understood that an unlimited number of configurations for the present invention could be realized. The foregoing discussion describes merely exemplary embodiments illustrating the principles of the present invention, the scope of which is recited in the following claims. Those skilled in the art will readily recognize from the description, claims, and drawings that numerous changes and modifications can be made without departing from the scope of the invention.

## Claims

1. A medical filter for therapeutic treatment of a patient, comprising:
first and second ends defining a longitudinal axis; and
a plurality of struts extending between the first and second ends, the struts tending to resiliently expand in radially outward directions from a compressed initial shape to an expanded deployed shape;
wherein in the expanded deployed shape, the struts define first and second filter sections and a center section connecting the filter sections; and
wherein the struts of each of the first and second filter sections define a number of filter cells, **characterised in that** an increased number of struts of the second filter section define a greater number of filter cells than the first filter section such that the second filter section exhibits a greater filtering efficiency than the first filter section.

2. The filter of Claim 1, adapted for use in a body passage or vessel defining a fluid flow direction, such that the first filter section is positioned upstream of the second filter section.

3. The filter of Claim 1, wherein the filter is formed out of one single unitary metal element.

4. The filter of Claim 1, adapted for use in the vena cava.

5. The filter of Claim 1, further comprising anchors formed on at least one surface of the vascular filter, adapted to increase position retention of the filter.

6. The filter of Claim 1, wherein in the expanded shape, a central portion of each strut tends to extend parallel to the longitudinal axis.

7. The filter of Claim 1, wherein the filter is made of nitinol.

## Patentansprüche

1. Ein medizinischer Filter für die therapeutische Behandlung eines Patienten, umfassend:
erste und zweite Enden, die eine Längsachse definieren; sowie eine Vielzahl an Streben bzw. Stützen zwischen den ersten und zweiten Enden, wobei sich die Streben bzw. Stützen tendenziell elastisch strahlenförmig nach außen ausdehnen, von einer komprimierten ursprünglichen Form zu einer ausgedehnten genutzten Form;
wobei in der ausgedehnten genutzten Form die Streben die ersten und zweiten Filterabschnitte definieren sowie ein Mittelteil die Filterabschnitte miteinander verbindet; und
wobei die Streben sowohl der ersten als auch der zweiten Filterabschnitte mehrere Filterzellen definieren, **dadurch gekennzeichnet, dass** eine größere Anzahl an Streben des zweiten Filterabschnitts eine größere Anzahl an Filterzellen definiert als der erste Filterabschnitt, so dass der zweite Filterabschnitt eine höhere Filtereffizienz als der erste Filterabschnitt aufweist.

2. Der Filter nach Anspruch 1, angepasst für die Benutzung in einem körpereigenen Durchgang oder Gefäß, die eine Fluidströmungsrichtung definieren, so dass der erste Filterabschnitt vor dem zweiten Filterabschnitt vorgelagert ist.

3. Der Filter nach Anspruch 1, wobei der Filter aus einem einzigen einheitlichen Metallbauteil besteht.

4. Der Filter nach Anspruch 1, angepasst für die Benutzung in der vena cava.

5. Der Filter nach Anspruch 1, der zusätzlich Anker enthält, die auf mindestens einer Oberfläche des vaskulären Filters gebildet werden, zur Verbesserung der Positionsretention des Filters.

6. Der Filter nach Anspruch 1, wobei sich in der ausgedehnten Form ein mittlerer Teil jeder Strebe bzw. Stütze tendenziell parallel zur Längsachse ausdehnt.

7. Der Filter nach Anspruch 1, wobei der Filter aus Nitinol besteht.

## Revendications

1. Filtre médical pour le traitement thérapeutique d'un patient, comprenant :
des première et seconde extrémités définissant un axe longitudinal ; et
une pluralité de supports s'étendant entre les première et seconde extrémités, les supports tendant à s'expanser de manière élastique dans des directions radialement vers l'extérieur d'une forme initiale comprimée à une forme déployée expansée ;
dans lequel dans la forme déployée expansée, les supports définissent des première et seconde sections de filtre et une section centrale reliant les sections de filtre ; et
dans lequel les supports de chacune des première et seconde sections de filtre définissent un nombre de cellules de filtre, **caractérisé en ce qu'**un nombre accru de supports de la seconde section de filtre définissent un nombre supérieur de cellules de filtre que la première section de filtre de telle sorte que la seconde section de filtre présente une efficacité de filtrage supérieure à celle de la première section de filtre.

2. Filtre selon la revendication 1, adapté pour être utilisé dans un passage ou vaisseau corporel définissant une direction d'écoulement de fluide, de telle sorte que la première section de filtre est positionnée en amont de la seconde section de filtre.

3. Filtre selon la revendication 1, dans lequel le filtre est formé d'un seul élément métallique unitaire.

4. Filtre selon la revendication 1, adapté pour être utilisé dans la veine cave.

5. Filtre selon la revendication 1, comprenant en outre des ancres formées sur au moins une surface du filtre vasculaire, conçues pour accroître le maintien en position du filtre.

6. Filtre selon la revendication 1, dans lequel dans la forme expansée, une partie centrale de chaque support tend à s'étendre parallèlement à l'axe longitudinal.

7. Filtre selon la revendication 1, le filtre étant constitué de nitinol.
